# EUROPEAN PATENT SPECIFICATION

(11) **EP 4 201 964 B1**
(45) Date of publication and mention of the grant of the patent: **27.11.2024**
(21) Application number: 21216347.1
(22) Date of filing: 21.12.2021
(51) Int. Cl.: C08B 30/18, A23L 29/25, A61K 9/20

(54) **PROCESS FOR THE PRODUCTION OF A LOW-VISCOSITY STARCH PRODUCT, THE PRODUCT OBTAINABLE BY THE PROCESS AND ITS USE**
VERFAHREN ZUR HERSTELLUNG EINES NIEDRIGVISKOSEN STÄRKEPRODUKTES, DAS NACH DEM VERFAHREN ERHÄLTLICHE PRODUKT UND SEINE VERWENDUNG
PROCÉDÉ DE PRODUCTION D'UN PRODUIT D'AMIDON À FAIBLE VISCOSITÉ, LE PRODUIT POUVANT ÊTRE OBTENU SELON CE PROCÉDÉ ET SON UTILISATION

(43) Date of publication of application: 28.06.2023
(73) Proprietor: Südstärke GmbH, 86529 Schrobenhausen (DE)
(72) Inventor: FRANK, Thomas, 86529 Schrobenhausen (DE)
(74) Representative: Hoefer & Partner Patentanwälte mbB

(56) References cited:
- WO-A1-98/56827
- JP-A- H01 287 101
- US-A1- 2020 385 494
- SHAMEKH S ET AL: "FILM FORMATION PROPERTIES OF POTATO STARCH HYDROLYSATES", STARCH/STARKE, WILEY-VCH VERLAG, WEINHEIM, DE, vol. 54, no. 1, 1 January 2002 (2002-01-01), pages 20 - 24, XP001092440, ISSN: 0038-9056, DOI: 10.1002/1521-379X(200201)54:1<20::AID-STAR20>3.0.CO;2-M
- WANG Y-J ET AL: "STRUCTURES AND PROPERTIES OF COMMERCIAL MALTODEXTRINS FROM CORN, POTATO, AND RICE STARCHES", STARCH/STARKE, WILEY-VCH VERLAG, WEINHEIM, DE, vol. 52, no. 8/09, 1 September 2000 (2000-09-01), pages 296 - 304, XP000959364, ISSN: 0038-9056, DOI: 10.1002/1521-379X(20009)52:8/9<296::AID-STAR296>3.0.CO;2-A
- YAN XIANG ET AL: "The combined effects of extrusion and heat-moisture treatment on the physicochemical properties and digestibility of corn starch", INTERNATIONAL JOURNAL OF BIOLOGICAL MACROMOLECULES, vol. 134, 20 May 2019 (2019-05-20), pages 1108 - 1112, XP085722951, ISSN: 0141-8130, DOI: 10.1016/J.IJBIOMAC.2019.05.112

## Description

### Field of the Invention

The present invention concerns a process for the production of a low-viscosity starch product and a product obtainable by the process. In the process, starch is first subjected to a specific heat-moisture treatment or a specific acid-heat treatment, and thereafter to an extrusion process in an extruder in order to obtain a (ultra) low-viscosity starch product. The invention is also concerned with the (ultra) low-viscosity, cold water-soluble starch products obtainable by the process.

### Technical Background

The global market demands a growing amount of starch products for various applications. Drivers for this market dynamic in the food segment is an increasing consumption of ready-to-eat meals and drinks as well as an upcoming trend for vegetarian food. To guarantee the starches' abilities to act as thickener or as gelling agent, chemical modifications have been established to maintain the stability during food processing, e.g. against low pH, heat treatment, shear forces and freeze-/thaw cycles. However, in addition to the solely thickening effect, starch products have to fulfil versatile functions in foods including volume expander, adsorbent properties for oils, spices and flavours, texturizer, structure and mouthfeel enhancer, carrier material, moisture regulator, film-forming agents, flowing aid in dry mixes, etc. However, a majority of starch products is not suitable for these applications because of the accompanying thickening effect especially when starch products were used in higher concentrations. Hence, there is a demand for low viscosity starch products in the food industry.

Oxidized starches used as food additive are produced by means of chemical modification. The use of sodium hypochlorite in a starch slurry results in low molecular weight starches labelled in the European Union under the E number 1404. Major disadvantages are high consummation of chemicals, emergence of wastewater and labelling issues for chemically modified starches.

White and yellow dextrins are obtained by dry roasting of acidified starch. On a chemical basis, the dextrinization is accompanied by hydrolysis, transglycosidation and repolymerization reaction, whereas the starch kernels remain intact (crystallinity of starch granules is reduced). Commercial available white dextrins are not 100% cold water-soluble (CWS). Yellow dextrins show a higher CWS compared to white dextrins but may be also limited in food use by solubility issues and additionally by their coloring.

Maltodextrins are obtained through partial hydrolysis of starch from crops like wheat, potatoes and maize. In short, the cold water-soluble maltodextrins are typically produced by i) preparation of starch solution, ii) acid-/ or enzymatic conversion, iii) inactivation of enzymes and iv) spray-drying procedure. Maltodextrins are used for manifold reasons in foods, especially in dry food applications but also in the beverage industry. They comprise several functional properties like texturizing, gelling, emulsifying abilities, flowability enhancer, and filling and adhering ingredient in dry mixtures. Moreover, maltodextrins have been described to be suitable to partly replace fat and gelatine from food recipes.

Although the functional properties of maltodextrins are undisputed, there is a deficit in perception from a consumer's perspective. In addition, production of maltodextrins is cost-intensive and plant-specifically challenging because of the spray-drying process used in the conventional process. Maltodextrins can be classified by the dextrose equivalent (DE) value, expressing the content of reducing sugars. Starch conversion products exhibiting DE levels between 3 and 20 are claimed to be maltodextrins. DE levels between 20 and 100 describe glucose syrup, a DE of 100 equals to pure glucose and DE levels below 3 are claimed to be starch (Starch Europe). Processes for enzyme-related maltodextrin production have been widely described in literature and were subjects for patent applications (e.g. WO2006/047176A).

A physical modification of starch is heat moisture treatment (HMT). This clean label treatment procedure has been developed in the late 1970th (e.g. DE2930664A). The treatment increases the gelatinization temperature making the cock-up starch suitable to being applied as a thickener in boiling soups and sauces without formation of lumps. In addition, heat moisture treatment of starches aims in the generation of high viscous starch products, in order to maintain the thickening performance. Especially after temperature breakdown, HMT starches show an increase in viscosity leading to comparable characteristics as chemically modified cross-linked starches. Thus, HMT starches are often being applied in catering systems.

JP H01 287101 A (JAPAN MAIZE PROD) 17 November 1989 discloses extrusion of starch in acidic medium.

### Summary of the Invention

A first aspect of the present invention concerns a process for the production of a low-viscosity starch product comprising the following first step and (subsequent) second step:
a) in the first step, native starch is subjected to
   a1) an acid-heat treatment wherein
      - the native starch is adjusted to a moisture content of 19 to 21 wt.-%,
      - 0.5 to 3 wt.-% of a diluted aqueous acid solution are added, wherein the acid concentration is 4 to 10 wt.-%, based on the total weight of the aqueous acid solution, in order to obtain an acid treated starch,
      - drying the acid-treated starch to a moisture content of below 6 wt.-%, based on the total weight of the acid-treated starch,
      - heating to a temperature within the range of 105 °C to 180 °C for a time period of 10 to 150 min,
      - adjusting the moisture content to 12 to 20 wt.-%, based on the total weight of the acid-heat treated starch, or
   a2) a heat-moisture treatment at a temperature of 105 °C to 120 °C for a time period of more than 10 hours at a moisture content of the native starch of 21 to 29 wt.-%, based on the total weight of the native starch, followed by adjusting the moisture content to 16 to 20 wt.-%, based on the total weight of the heat-moisture treated starch,
   in order to obtain an intermediate starch product having a content of reducing sugars of less than 3 wt.-%, based on the total weight of the intermediate starch product, and a cold water solubility of less than 90 %, in particular less than 50 %,
b) in the second step, subsequent to the first step, the intermediate starch product is subjected to an extrusion process in an extruder, preferably a twin screw extruder, in order to obtain the low-viscosity starch product, wherein the low-viscosity starch product has an end viscosity measured according to the Brabender method of less than 50 BU, a content of reducing sugars of less than 3 wt.-%, and a cold water solubility of 100 wt.-%.

A further aspect of the present invention concerns a low-viscosity starch product obtainable according to the process above or described further herein. The low-viscosity starch product of the invention has a content of reducing sugars of less than 3 wt.-% (measured according to Luff-Schoorl), and a cold water solubility of 100 wt.-%. The low-viscosity starch product of the invention has an end viscosity of less than 50 BU, in particular less than 40 BU (measured according to standardized Brabender method).

Yet a further aspect of the present invention concerns the use of the low-viscosity starch product described herein, obtainable according to the process above or described further herein, in a nutritional, pharmaceutical of veterinary composition, in particular as a substitute for maltodextrin.

### Detailed Description of the Invention

The inventors have found that the process of the invention as described herein surprisingly allows the production of a very low viscosity starch product without chemical modification in a convenient and economic way. The starch products according to the invention, obtainable by the process of the invention, have advantageous properties comparable to or even superior to maltodextrins, without requiring the more cost- or energy-consuming steps of maltodextrin preparation according to the prior art. The starch products according to the invention are even superior to prior art starch products in their functional properties like texturizing, gelling, emulsifying abilities, use as flowability enhancer, filling and adhering ingredients in dry mixtures, and partial replacements for fat and gelatine in food recipes. They have a pure white colour and excellent flowability properties. The starch products according to the invention do not require a classification as maltodextrins in Europe.

It has been found that the pre-treatment of a starch (see steps a1 and a2 above), in particular a native starch, as specified in the process described herein, advantageously enables preparation of an intermediate starch product which can conveniently be converted into a low starch product by conventional extrusion in an extruder as known in the art. The final product of the process according to the invention advantageously provides cold water solubility, very low end viscosity as defined herein, at least comparable to conventional maltodextrins, and a very low content of reducing sugars, below the conventional maltodextrins of the prior art. From a producer's prospective, it provides alternative products to maltodextrins and their production processes, saving production-related resources, waste streams and costs, and providing even superior properties.

The term "low viscosity starch product" as used herein describes a starch product which provides properties corresponding to and even superior to commercial maltodextrins without being prepared in accordance with the prior art preparation of maltodextrins, and not requiring in Europe labelling as maltodextrins. Low viscosity, sometimes called very low viscosity or ultra-low viscosity herein, shall preferably designate a starch or starch product having an end viscosity of less than 50 BU, in particular less than 40 BU, more particular less than 35 BU. The determination of Brabender viscosity is known to the skilled person. Any Brabender viscograph known in the art can be used for determining the Brabender viscosity as used herein. Preferably, the Brabender viscosity is measured according to a standardized Brabender method as described in Methods below. Moreover, the content of reducing sugars of the low-viscosity starch product of the invention is less than 3 wt.-%, in particular less than about 2.5 wt.-%, more particular at most 2 wt.-%. It has surprisingly been found that the advantage of such a low content of reducing sugar may be obtained by the process of the invention as described herein, offering advantages regarding the usability and calorimetric properties while maintaining the (ultra-)low viscosity of conventional maltodextrins, and their cold water solubility. Thus, the low viscosity starch products of the present invention also provide a cold water solubility of 100 wt.-%.

In the first step of the process of the invention, starch, in particular native (natural) starch, is used. The native starch is preferably not chemically modified. Waxy starches and high amylose starches, i.e. (chemically unmodified) starches with a very high amylopectin content or a very high amylose content are also considered within the term native starch as used herein. The starch may be obtained from any starch-producing plant, in particular potato starch, maize starch, corn starch, starch from tapioca, arrowroot, wheat, rice, sago, mung bean or the like. Potato starch is particularly preferred.

In the first step of the process of the invention, the (native) starch is subjected to a heat-moisture treatment, i.e. a combination of heat and a specific moisture content of the starch is used for this treatment step.

Heat moisture treatment (HMT) in the prior art is a physical methodology for starch processing aiming at achieving highly viscous sauce thickeners exhibiting elevated gelatinization temperature. However, the present invention relates to the use of an overexpressed heat moisture treatment leading to a significant reduction of starch viscosity. Although not desired for the production of regular HMT starch used as sauce thickener, surprisingly, overexpressed HMT starch (called "intermediate starch product" in the process of the invention) has been shown to be a very suitable feeding material for processing by means of extrusion in order to obtain a very advantageous final starch product as defined herein. To obtain overexpressed HMT starch, (native) starch is treated under "harsh" conditions regarding temperature and treatment time.

It has been found that a temperature of at least about 105 °C is required in order to obtain satisfactory results of the treatment, preparing the resulting starch product (called intermediate starch product herein) for the subsequent extrusion step. Preferably, the temperature should be at most about 125 °C, in particular about 120 °C. According to a particular preferred embodiment of the present invention, the temperature used in the first step, namely the heat-moisture treatment, is from about 105 °C to about 120 °C.

Further, it is required that the heat-moisture treatment, is performed for a time period of more than about 10 hours at a moisture content of the starch of about 21 to about 29 wt.-%, based on the total weight of the (native) starch. The HMT is preferably performed in a tightly closed vessel.

Surprisingly, the present inventors have found that, together with the temperature of the HMT as specified above, this minimum time of about 10 hours together with the particular moisture content as specified above is required in order to obtain a physical modification of the starch preparing it advantageously for the subsequent extrusion step when aiming at a very low viscosity starch product as specified herein.

Thus, it has been found that a moisture content of the (native) starch of below 21 wt.-% as well as a too high moisture content above 29 wt.-%, based on the total weight of the (native) starch does not impact the starch in a reliable and advantageous way, and does not prepare it sufficiently for subsequent extrusion, aiming at the low viscosity starch product as described herein, in particular having full cold water-solubility, very low or ultra-low viscosity, no viscosity peak when heating, and a content of reducing sugars of less than 3%. Preferably, native starch is adjusted to a water (moisture) content of about 22 to about 25% by weight, based on the total weight of the (native) starch, for the HMT.

Subsequent to the HMT as described above (but before the extrusion step), the moisture content of the starch is slightly lowered to a range from about 16 to about 20 wt.-%, based on the total weight of the heat-moisture treated starch. It has been found that such a slight reduction of moisture or drying of the starch after the HMT is advantageous for an efficient extrusion when aiming at the low viscosity starch product as described herein.

It has further been found that, as an alternative to the HMT as the first step of the process of the invention, also an acid-heat treatment, also called AHT, may be performed.

According to this alternative of the inventive process, the (native) starch is adjusted to a moisture content of about 19 to 21 wt.-%. At this moisture content, about 0.5 to about 3 wt.-%, in particular about 1 to about 2 wt.-% of a diluted aqueous acid solution (based on the total weight of the starch used for the AHT) are added, wherein the acid concentration is 4 to 10 wt.-%, based on the total weight of the aqueous acid solution, in order to obtain an acid-treated starch. Any acid conventional in the art may be used. Hydrochloric acid (HCl) is preferred. The acidification is performed for about 5 minutes to about 1 hour. The exact time is not critical and is normally achieved within short time. The starch, having the moisture content as adjusted above, and the diluted aqueous acid solution are usually mixed in order to allow a homogenous mixture of the acid with the starch. The mixing is preferably carried out for (at least) several minutes. Room temperature or a temperature different from room temperature may be used for adding the diluted aqueous acid solution and/or mixing.

Subsequently, the acid-treated starch is dried to a moisture content of below about 6 wt.-%, in particular about 1 to 6 wt.-%, based on the total weight of the acid-treated starch, and then heated to a temperature within the range of about 105 °C to about 180 °C, preferably 120 to 180°C, more preferably 130 to150°C, for a time period of about 10 to about 50 minutes. Preferably, treatment time is 10 to 30 minutes. More preferably, treatment time is 15 to 20 minutes. Preferably, the starch is stirred during heat treatment at the adjusted temperature level.

Diverging from the aforementioned conditions of the AHT will pose a risk that feeding the resulting material in the extruder causes considerable impairments of the extrusion process, e.g. blocking of barrels or other extrusion processing problems.

After the heating step (in the ATH), the resulting material will be preferably cooled to a certain temperature, ideally ambient temperature. Also, the moisture content is adjusted to about 12 to about 20 wt.-%, in particular about 12 to about 15 wt.-%, based on the total weight of the acid-heat treated starch. It has further been found advantageous to (partially) achieve this adjustment in the moisture content by allowing the (dry) acid-heat treated starch to cool in an open atmosphere, absorbing moisture from the surrounding atmosphere (for the subsequent extrusion step).

It has been found that, as an alternative to the specific HMT as described above, the AHT with the steps as specified above allows obtaining an immediate starch product which is particularly suitable for subsequent conventional extrusion in order to obtain a low viscosity starch product as described herein.

In general, the intermediate starch product has a content of reducing sugars of less than 3 wt.-%, in particular less than about 2.5 wt.-%, more particular less than about 2 wt.-%. Determination of the content of reducing sugars is known to the person skilled in the art and can for example be measured as indicated in the Method section below. Furthermore, the intermediate starch product has a cold water solubility of less than 90 %, in particular less than 50 %, preferably in the range of 2 to 25 %, more preferably in the range of about 5 to 15%. The cold water solubility can be measured as indicated in the section Methods below.

Preferably, the intermediate starch product obtained after the first step (a1 or a2) of the process of the invention has a viscosity of less than 500 cp according to RVA at 50°C. The RVA viscosity as specified above can be determined as known in the art. Preferably, it is measured in a standardized RVA method as described in the Methods Section below.

All indications of percentage as used herein are wt.-% based on the total weight of the composition, unless indicated differently.

According to the process of the invention, the intermediate starch product obtained from the first step is subjected to a second step, wherein the intermediate starch product is subjected to an extrusion process in an extruder in order to obtain the low viscosity starch product as described herein. The specific process step of the first step according to the inventive process allow preparing an intermediate product which is particularly suitable for subsequent extrusion, using standard extrusion devices and parameters, in order to obtain the desired low-viscosity starch product of the invention.

Application of extrusion to native starch according to the prior art is limited to a certain degree of viscosity in the extruded starch product and has disadvantages regarding the impact on the properties of final extruded product and its functional utility. An overexertion of extrusion conditions which may lead to production issues, such as blocking of barrels, an off-white colouring or partly burned starch, or non-desirable fluctuations of bulk-weight density in the end product is avoided by the present invention.

The present invention, combining a specific pre-treatment (first step) as described herein and subsequent extrusion allows to surprisingly obtain a low viscosity (CWS) starch product having the advantageous properties as described herein.

Standard extruder devices and parameters are known to the skilled person and can be used in the present invention. According to a preferred embodiment of the invention, a twin-screw extruder device may be used.

According to a preferred embodiment, during the heat-moisture treatment, the starch has a moisture content of 21 to 28 wt.-%, in particular 22 to 25 wt.-%, based on the total weight of the native starch. It has been found that this moisture content allows a particularly advantageous HMT.

As indicated above, in the prior art, a HMT process was used in order to delay and increase the viscosity development of a starch. In contrast, it has surprisingly been found by the present inventors that a HMT process, if the specific parameters as set out herein are followed, may be used in order to prepare an intermediate starch product not having an increased or delayed viscosity, but being adapted for transforming into a low viscosity starch product as described herein (having properties not only similar to but even superior to maltodextrins) by a simple extrusion process.

According to a further preferred embodiment of the invention, the HMT is performed at a temperature of 105 °C to 115 °C.

Also, preferably, the moisture content is adjusted to 17-19 wt.-%, based on the total weight of the heat-moisture treated starch, following the HMT.

As explained above, it has been found advantageous to adjust/lower the moisture content of the heat-moisture treated starch to 16 to 20 wt.-%. It has further been found advantageous to (partially) achieve this adjustment/reduction in the moisture content by allowing the moisture-heat treated starch to cool in an open atmosphere, removing moisture (for the subsequent extrusion step).

In a further preferred embodiment of the present invention, in the AHT, 1 to 2 wt.-% of a diluted aqueous acid solution, preferably HCl solution, are added to the (native) starch as described above.

According to a preferred embodiment of the present invention, during the extrusion in an extruder (second step of the process of the invention), the highest temperature in the extruder is about 130 to 180 °C if the intermediate starch product (used for extrusion) is obtained according to a HMT as described herein, and about 110 to 150 °C if the intermediate starch product is obtained according to the AHT as described herein.

The inventors have surprisingly found that the present process of the invention, combining a simple pre-treatment step (first step in the process according to the invention) with a convenient extrusion step allows simple and continuous production of a very advantageous low viscosity starch product.

According to a further aspect of the present invention, the low viscosity starch product of the invention as described herein may be advantageously used in a nutritional, pharmaceutical or veterinary composition. In particular, it may be used, partially or completely, as a replacement for maltodextrins in such compositions.

According to a preferred embodiment of the present invention, the nutritional composition may be a food or beverage composition, in particular a dry food or beverage composition.

A further preferred embodiment of the present invention pertains to the use of the low viscosity starch product of the invention (as described herein) as an emulsifying agent, a flowability-enhancing agent, a filling, binding or adhering agent. It has surprisingly been found that the low viscosity starch product of the present invention, as described herein, provides advantageous in relation to the above uses desirable for the skilled person in the art.

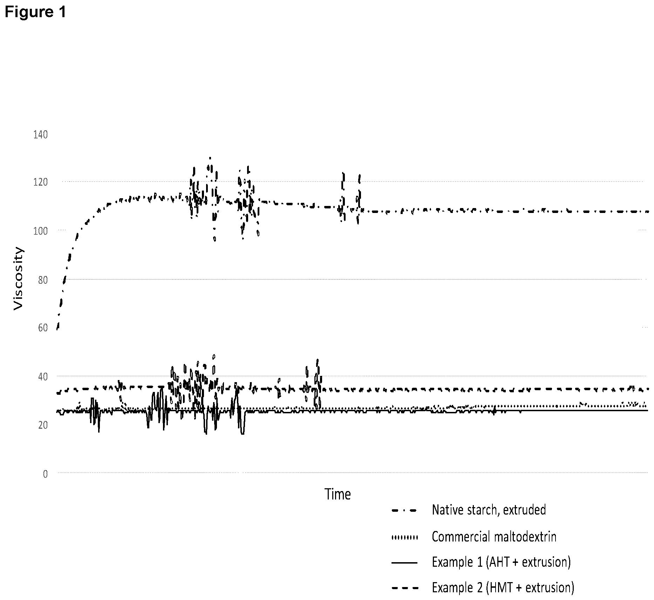

Figure 1 shows the viscosity curves (Brabender viscosity determined as described above) of low viscosity starch products according to the invention (Examples 1 and 2) in comparison to non-treated (native) extruded starch and a commercial maltodextrin.

### Methods:

### RVA (Rapid Visco Analyzer) Viscosity:

The RVA viscosity values as indicated herein can be measured in a standardized RVA method: RVA (Rapid Visco Analyzer Super-4, NewPort Scientific) was used for viscosity measurement. 2.5g dry starch was added to 25g distilled water and subjected to RVA analysis. The slurry was heated to 95°C followed by a cooling phase to 50°C where the end viscosity was determined. "Dry starch" shall mean that the material was dried to constant weight at 120°C in an oven (normally achieved after about 8-10 min).

### Brabender Units (BU):

This can be measured in a standardized Brabender Method: The viscosity of cold water-soluble low viscosity starch products were measured according to a customized Brabender method (Brabender Viscograph Type E, measuring range 250cmg, temperature gradient 25°C to 30°C) by mixing 30g dry starch to 55g ethylene glycol and 400g distilled water. End viscosity was measured at a temperature of 30°C. "Dry starch" shall mean that the material was dried to constant weight at 120°C in an oven (normally achieved after about 8-10 min).

### Cold water solubility of starch:

Determination of cold water solubility of starch is known in the art. According to a preferred embodiment, it can be performed according to the method of Höppler, see e.g. O. Wolff, "Die Stärke" 11, Seiten 273-279, 1950, Kapitel "Dextrin. Herstellung, Untersuchungs-methoden und Gütebestimmungen", Section 3. "Kaltwasserlöslichkeit" on page 276.

The cold water solubility of starch can alternatively be determined by measuring the refractive index of a starch solution. 10g of starch on dry weight basis are dispersed in 90g distilled water. The suspension is filtrated through a folded filter and the obtained solution is measured by means of a refractometer. The determined refractive index is directly correlated to the degree of cold water solubility. "On dry weight basis" shall mean that the material was dried to constant weight at 120°C in an oven (normally achieved after about 8-10 min).

### Content of reducing sugars:

The content of reducing sugars can be measured according to the method of Luff-Schoorl (see e.g. Reinhard Matissek, Gabriele Steiner, "Lebensmittelanalytik: Grundzüge, Methoden, Anwendungen." 3., vollst. überarb. Auflage, Springer Verlag, Berlin/Heidelberg 2006, ISBN 3-540-62513-5, pages 115 and following).

### Moisture content of starch (products):

The moisture or water contents in wt.-% as indicated herein are determined by drying the material to constant weight at 120°C in an oven and comparing the weight of the sample before and after drying.

### Examples

In the following, the invention will be further illustrated by the following examples.

### Example 1: AHT (acid-heat treatment) of native starch, followed by extrusion

A diluted aqueous solution of hydrochloric acid (1 wt.-%) was sprayed on native potato starch and intensively mixed. After conditioning at ambient temperature for 15 minutes, the HCl-treated starch was dried to a water content less than 4%. Immediately after that, the starch was transferred to a pan and roasted for 15 minutes at 140°C. The resulting material was then rehydrated to 13 wt.-% moisture content (partially by allowing the heated starch after the roasting to absorb moisture from the environment) and exhibited a cold water-solubility (CWS) of 7%, a molecular weight of less than 100.000 g/mol, and a content of reducing sugars below 1%. It had a viscosity of 20 cp according to RVA at 50°C, measured as indicated above (Methods).

The pre-treated starch (intermediate starch product) was used as feeding material (variable feeding rate 400 to 750kg/h) in a co-rotating Werner & Pfleiderer twin screw extruder (screw speed 280 rpm) equipped with two reverse elements, a volumetric feeding system, and temperature-controlled casing blocks. Standard parameters as indicated by the manufacturer were used for extrusion.

### Example 2: HMT (heat-moisture treatment) of native starch, followed by extrusion

Native potato starch was moistened to a water content of 22% and transferred to tightly closed vessels. After incubation for 14 hours at 110°C, the HMT starch was cooled down to ambient temperature in open atmosphere so that moisture could evaporate, and adjusted to a moisture content of 18%.

The pre-treated starch (intermediate starch product) was used as feeding material for a standard extrusion as set out in Example 1 above.

For comparison, native potato starch (not pre-treated by AHT or HMT) conventionally extruded under conditions as used in Examples 1 and 2, and a commercially available maltodextrin were used.

The low-viscosity starch product samples obtained from Examples 1 and 2 and the comparative samples were ground to a milling degree < 200 µm and used for viscosity determination. Figure 1 shows the respective viscosity curves for the starch product of the invention obtained from Examples 1, Example 2, extruded native starch (comparison) and commercial maltodextrin (comparison), at a concentration of 10 wt.-% in distilled water. Measurement of reducing sugars in the described starch samples revealed contents of less than 3% (Example 1: 2%, Example 2: 0.4%, extruded native starch: <0.2%, used commercial maltodextrin: 3%). It is evident from Figure 1 that the starch products according to the invention had a low viscosity even comparable to or better than the commercial maltodextrin, while having a white colour, excellent followability and low level of reducing sugars.

## Claims

1. Process for the production of a low-viscosity starch product comprising the following first step and subsequent second step:
a) in the first step, native starch is subjected to
a1) an acid-heat treatment wherein
- the native starch is adjusted to a moisture content of 19 to 21 wt.-%,
- 0.5 to 3 wt.-% of a diluted aqueous acid solution are added, wherein the acid concentration is 4 to 10 wt.-%, based on the total weight of the aqueous acid solution, in order to obtain an acid treated starch,
- drying the acid-treated starch to a moisture content of below 6 wt.-%, based on the total weight of the acid-treated starch,
- heating to a temperature within the range of 105 °C to 180 °C for a time period of 10 to 150 min,
- adjusting the moisture content to 12 to 20 wt.-%, based on the total weight of the acid-heat treated starch, or
a2) a heat-moisture treatment at a temperature of 105 °C to 120 °C for a time period of more than 10 hours at a moisture content of the native starch of 21 to 29 wt.-%, based on the total weight of the native starch, followed by adjusting the moisture content to 16 to 20 wt.-%, based on the total weight of the heat-moisture treated starch,
in order to obtain an intermediate starch product having a content of reducing sugars of less than 3 wt.-%, based on the total weight of the intermediate starch product, and a cold water solubility of less than 90%, in particular less than 50 %,
b) in the second step, subsequent to the first step, the intermediate starch product is subjected to an extrusion process in an extruder, preferably a twin screw extruder, in order to obtain the low-viscosity starch product, wherein the low-viscosity starch product has an end viscosity measured according to the Brabender method of less than 50 BU, a content of reducing sugars of less than 3 wt.-%, and a cold water solubility of 100 wt.-%.

2. Process according to claim 1, wherein during the heat-moisture treatment according to a2) the native starch has a moisture content of 21 to 28 wt.-%, in particular 22 to 25 wt.-%, based on the total weight of the native starch.

3. The process according to any of the preceding claims, wherein the heat-moisture treatment according to a2) is performed at a temperature of 105 °C to 115 °C.

4. The process according to any of the preceding claims, wherein in step a2) following the moisture-heat treatment, the moisture content is adjusted to 17 - 19 wt.-%, based on the total weight of the heat-moisture treated starch.

5. The process according to any of the preceding claims, wherein the adjusting of the moisture content to 16 to 20 wt.-% according to a2) involves allowing the moisture-heat treated starch to cool in an open atmosphere, removing moisture for the subsequent extrusion step b).

6. The process according to any of the preceding claims, wherein the intermediate starch product has a RVA viscosity of less than 500 cp measured at 10 wt.-% in distilled water at 50°C.

7. The process according to any of the preceding claims, wherein the low-viscosity starch product has an end viscosity measured according to the Brabender method of less than 40 BU.

8. The process according to any of the preceding claims, wherein the native starch is a native potato starch.

9. The process according to any of the preceding claims, wherein 1 to 2 wt.-% of a diluted aqueous acid solution are added in step a1).

10. The process according to any of the preceding claims, wherein the highest temperature in the extruder is 130 to 180 °C if the intermediate starch product is obtained according to a2), and 110 to 150 °C if the intermediate starch product is obtained according to a1).

11. Low-viscosity starch product obtainable according to the process according to any one of claims 1 to 10, having an end viscosity, measured according to standardized Brabender method of less than 50 BU, in particular less than 40 BU, a content of reducing sugars, measured according to Luff-Schoorl of less than 3 wt.-%, and a cold water solubility of 100 wt.-%.

12. Use of a low-viscosity starch product according to claim 11 or obtained by a process according to any one of claims 1 to 9 as a substitute for maltodextrin in a nutritional, pharmaceutical of veterinary composition.

13. Use according to claim 12 in a food or beverage composition, in particular in a dry food or beverage.

14. Use according to one of claims 12 or 13 as an emulsifying agent, a flowability-enhancing agent, a filling, binding or adhering agent.

## Patentansprüche

1. Verfahren zur Herstellung eines niedrigviskosen Stärkeproduktes umfassend den folgenden ersten Schritt und anschließenden zweiten Schritt:
a) wobei in dem ersten Schritt native Stärke
a1) einer Säure/Wärme-Behandlung, wobei
- die native Stärke auf einen Feuchtigkeitsgehalt von 19 bis 21 Gew.-% eingestellt wird,
- 0,5 bis 3 Gew.-% von einer verdünnten wässrigen Säurelösung zugegeben werden, wobei die Säurekonzentration 4 bis 10 Gew.-%, bezogen auf das Gesamtgewicht der wässrigen Säurelösung, beträgt, um eine säurebehandelte Stärke zu erhalten,
- Trocknen der säurebehandelten Stärke auf einen Feuchtigkeitsgehalt von unter 6 Gew.-%, bezogen auf das Gesamtgewicht der säurebehandelten Stärke,
- Erwärmen auf eine Temperatur innerhalb des Bereichs von 105 °C bis 180 °C für eine Zeitdauer von 10 bis 150 min,
- Einstellen des Feuchtigkeitsgehalts auf 12 bis 20 Gew.-%, bezogen auf das Gesamtgewicht der Säure/Wärme-behandelten Stärke, oder
a2) einer Wärme/Feuchtigkeit-Behandlung bei einer Temperatur von 105 °C bis 120 °C für eine Zeitdauer von länger als 10 Stunden bei einem Feuchtigkeitsgehalt der nativen Stärke von 21 bis 29 Gew.-%, bezogen auf das Gesamtgewicht der nativen Stärke, gefolgt von Einstellen des Feuchtigkeitsgehalts auf 16 bis 20 Gew.-%, bezogen auf das Gesamtgewicht der Wärme/Feuchtigkeit-behandelten Stärke,
unterworfen wird,
um ein Stärke-Zwischenprodukt mit einem Gehalt an reduzierenden Zuckern von niedriger als 3 Gew.-%, bezogen auf das Gesamtgewicht des Stärke-Zwischenprodukts, und einer Kaltwasser-Löslichkeit von niedriger als 90%, insbesondere niedriger als 50 % zu erhalten,
b) wobei in dem zweiten Schritt, anschließend an den ersten Schritt, das Stärke-Zwischenprodukt einem Extrusionsverfahren in einem Extruder, bevorzugt einem Doppelschneckenextruder unterworfen wird,
um das niedrigviskose Stärkeprodukt zu erhalten, wobei das niedrigviskose Stärkeprodukt eine Endviskosität gemessen gemäß dem Brabender-Verfahren von niedriger als 50 BU, einen Gehalt an reduzierenden Zuckern von niedriger als 3 Gew.-%, und eine Kaltwasser-Löslichkeit von 100 Gew.-% aufweist.

2. Verfahren gemäß Anspruch 1, wobei während der Wärme/Feuchtigkeit-Behandlung gemäß a2) die native Stärke einen Feuchtigkeitsgehalt von 21 bis 28 Gew.-%, insbesondere 22 bis 25 Gew.-%, bezogen auf das Gesamtgewicht der nativen Stärke, aufweist.

3. Verfahren gemäß einem der vorhergehenden Ansprüche, wobei die Wärme/Feuchtigkeit-Behandlung gemäß a2) bei einer Temperatur von 105 °C bis 115 °C durchgeführt wird.

4. Verfahren gemäß einem der vorhergehenden Ansprüche, wobei in Schritt a2) folgend der Feuchtigkeit/Wärme-Behandlung der Feuchtigkeitsgehalt auf 17 - 19 Gew.-%, bezogen auf das Gesamtgewicht der Wärme/Feuchtigkeit-behandelten Stärke, eingestellt wird.

5. Verfahren gemäß einem der vorhergehenden Ansprüche, wobei das Einstellen des Feuchtigkeitsgehalts auf 16 bis 20 Gew.-% gemäß a2) Ermöglichen, dass die Feuchtigkeit/Wärme-behandelte Stärke in einer offenen Atmosphäre abkühlt, Entfernen von Feuchtigkeit für den anschließenden Extrusionsschritt b) einbezieht.

6. Verfahren gemäß einem der vorhergehenden Ansprüche, wobei das Stärke-Zwischenprodukt eine RVA-Viskosität von niedriger als 500 cp gemessen bei 10 Gew.-% in destilliertem Wasser bei 50°C aufweist.

7. Verfahren gemäß einem der vorhergehenden Ansprüche, wobei das niedrigviskose Stärkeprodukt eine Endviskosität gemessen gemäß dem Brabender-Verfahren von niedriger als 40 BU aufweist.

8. Verfahren gemäß einem der vorhergehenden Ansprüche, wobei die native Stärke eine native Kartoffelstärke ist.

9. Verfahren gemäß einem der vorhergehenden Ansprüche, wobei in Schritt a1) 1 bis 2 Gew.-% von einer verdünnten wässrigen Säurelösung zugegeben werden.

10. Verfahren gemäß einem der vorhergehenden Ansprüche, wobei die höchste Temperatur in dem Extruder 130 bis 180 °C, wenn das Stärke-Zwischenprodukt gemäß a2) erhalten wird, und 110 bis 150 °C, wenn das Stärke-Zwischenprodukt gemäß a1) erhalten wird, beträgt.

11. Niedrigviskoses Stärkeprodukt erhältlich gemäß dem Verfahren gemäß einem der Ansprüche 1 bis 10, mit einer Endviskosität gemessen gemäß einem standardisierten Brabender-Verfahren von niedriger als 50 BU, insbesondere niedriger als 40 BU, einem Gehalt an reduzierenden Zuckern gemessen gemäß Luff-Schoorl von niedriger als 3 Gew.-%, und einer Kaltwasser-Löslichkeit von 100 Gew.-%.

12. Verwendung eines niedrigviskosen Stärkeproduktes gemäß Anspruch 11 oder erhalten durch ein Verfahren gemäß einem der Ansprüche 1 bis 9 als ein Ersatz für Maltodextrin in einer Ernährungs-, pharmazeutischen oder Veterinärzusammensetzung.

13. Verwendung gemäß Anspruch 12 in einer Lebensmittel- oder Getränkezusammensetzung, insbesondere in einem trockenen Lebensmittel oder Getränk.

14. Verwendung gemäß einem der Ansprüche 12 oder 13 als ein Emulgiermittel, ein Fließfähigkeit-steigerndes Mittel, ein Füllstoff, Bindemittel oder haftendes Mittel.

## Revendications

1. Procédé de production d'un produit d'amidon à faible viscosité comprenant la première étape et la deuxième étape consécutive qui suivent :
a) dans la première étape, l'amidon natif est soumis à
a1) un traitement thermique acide dans lequel
- l'amidon natif est ajusté à une teneur en humidité de 19 à 21% en poids,
- 0,5 à 3% en poids d'une solution acide aqueuse diluée sont ajoutés, dans lequel la concentration en acide est de 4 à 10% en poids, par rapport au poids total de la solution acide aqueuse, afin d'obtenir un amidon traité à l'acide,
- séchage de l'amidon traité à l'acide jusqu'à une teneur en humidité inférieure à 6% en poids, par rapport au poids total de l'amidon traité à l'acide,
- chauffage à une température dans la plage de 105°C à 180°C pendant une durée de 10 à 150 min,
- ajustement de la teneur en humidité entre 12 et 20% en poids, par rapport au poids total de l'amidon traité à l'acide, ou
a2) un traitement par chaleur et humidité à une température de 105°C à 120°C pendant une durée supérieure à 10 heures à une teneur en humidité de l'amidon natif de 21 à 29% en poids, par rapport au poids total de l'amidon natif, suivi d'un ajustement de la teneur en humidité entre 16 et 20% en poids, par rapport au poids total de l'amidon traité par chaleur et humidité,
afin d'obtenir un produit d'amidon intermédiaire ayant une teneur en sucres réducteurs inférieure à 3% en poids, par rapport au poids total du produit d'amidon intermédiaire, et une solubilité dans l'eau froide inférieure à 90%, en particulier inférieure à 50%,
b) dans la deuxième étape, consécutive à la première étape, le produit d'amidon intermédiaire est soumis à un processus d'extrusion dans une extrudeuse, de préférence une extrudeuse à double vis,
afin d'obtenir le produit d'amidon à faible viscosité, dans lequel le produit d'amidon à faible viscosité a une viscosité finale mesurée selon la méthode Brabender inférieure à 50 BU, une teneur en sucres réducteurs inférieure à 3% en poids et une solubilité dans l'eau froide de 100% en poids.

2. Procédé selon la revendication 1, dans lequel, pendant le traitement par chaleur et humidité selon a2), l'amidon natif présente une teneur en humidité de 21 à 28% en poids, en particulier de 22 à 25% en poids, par rapport au poids total de l'amidon natif.

3. Procédé selon l'une des revendications précédentes, dans lequel le traitement par chaleur et humidité selon a2) est effectué à une température de 105°C à 115°C.

4. Procédé selon l'une des revendications précédentes, dans lequel, à l'étape a2) après le traitement par chaleur et humidité, la teneur en humidité est ajustée entre 17 et 19% en poids, par rapport au poids total de l'amidon traité par chaleur et humidité.

5. Procédé selon l'une des revendications précédentes, dans lequel l'ajustement de la teneur en humidité entre 16 et 20% en poids selon a2) consiste à laisser l'amidon traité par chaleur et humidité refroidir dans une atmosphère ouverte, en éliminant l'humidité pour l'étape d'extrusion consécutive b).

6. Procédé selon l'une des revendications précédentes, dans lequel le produit d'amidon intermédiaire a une viscosité RVA inférieure à 500 cp mesurée à 10% en poids dans de l'eau distillée à 50°C.

7. Procédé selon l'une des revendications précédentes, dans lequel le produit d'amidon à faible viscosité a une viscosité finale mesurée selon la méthode Brabender inférieure à 40 BU.

8. Procédé selon l'une des revendications précédentes, dans lequel l'amidon natif est un amidon de pomme de terre natif.

9. Procédé selon l'une des revendications précédentes, dans lequel 1 à 2% en poids d'une solution acide aqueuse diluée sont ajoutés à l'étape a1).

10. Procédé selon l'une des revendications précédentes, dans lequel la température la plus élevée dans l'extrudeuse est de 130 à 180°C si le produit d'amidon intermédiaire est obtenu selon a2), et de 110 à 150°C si le produit d'amidon intermédiaire est obtenu selon a1).

11. Produit d'amidon à faible viscosité pouvant être obtenu selon le procédé selon l'une quelconque des revendications 1 à 10, ayant une viscosité finale, mesurée selon la méthode Brabender normalisée inférieure à 50 BU, en particulier inférieure à 40 BU, une teneur en sucres réducteurs, mesurée selon Luff-Schoorl inférieure à 3% en poids, et une solubilité dans l'eau froide de 100% en poids.

12. Utilisation d'un produit d'amidon à faible viscosité selon la revendication 11 ou obtenu par un procédé selon l'une quelconque des revendications 1 à 9 comme substitut de la maltodextrine dans une composition nutritionnelle, pharmaceutique ou vétérinaire.

13. Utilisation selon la revendication 12 dans une composition d'aliment ou de boisson, en particulier dans un aliment sec ou une boisson.

14. Utilisation selon l'une des revendications 12 ou 13 comme agent émulsifiant, agent améliorant la fluidité, agent de remplissage, liant ou adhésif.
